# EUROPÄISCHE PATENTANMELDUNG

(11) **EP 4 840 448 A1**
(43) Veröffentlichungstag der Anmeldung: **18.11.2026**
(21) Anmeldenummer: 25176495.7
(22) Anmeldetag: 14.05.2025
(51) Int. Cl.: A61K 31/357, A61K 31/4025, A61K 31/662, A61P 33/06

(54) **KOMBINATIONSPRÄPARATE VON 3-N-FORMYLHYDROXYLAMINOPROPYLPHOSPHONSÄUREDERIVATEN ODER 3-N-ACETYLHYDROXYLAMINOPROPYLPHOSPHONSÄUREDERIVATEN MIT CLINDAMYCIN UND ARTESUNAT FÜR DIE MALARIA-STAND-BY-MEDIKATION**

(71) Anmelder: OBB Oertel Beratungs- und Beteiligungsgesellschaft mbH, 22605 Hamburg (DE)
(72) Erfinder: Oertel, Frank Wilhelm, 22605 Hamburg (DE)
(74) Vertreter: Simandi, Claus

(57) **Zusammenfassung**

Die vorliegende Erfindung betrifft pharmazeutische Präparate, die als Wirkstoffe 3-N-Formylhydroxylaminopropylphosphonsaurederivate oder 3-N-Acetylhydroxylaminopropylphosphonsaurederivate in Kombination mit Clindamycin und Artesunat zur Behandlung von Malaria enthalten, und zwar für die Malaria-Stand-by-Medikation.

## Beschreibung

Die vorliegende Erfindung betrifft pharmazeutische Präparate, die als Wirkstoffe 3-N-Formylhydroxylaminopropylphosphonsaurederivate oder 3-N-Acetylhydroxylaminopropylphosphonsaurederivate in Kombination mit Clindamycin und Artesunat zur Behandlung von Malaria enthalten, und zwar für die Malaria-Stand-by-Medikation.

Die Verwendung von 3-N-Formylhydroxylaminopropylphosphonsaurederivaten und 3-N-Acetylhydroxylaminopropylphosphonsaurederivaten zur prophylaktischen und therapeutischen Behandlung von infektiösen Prozessen, insbesondere Infektionen, die durch einzellige Parasiten (im Sinne dieser Erfindung auch Protozoen genannt) oder mehrzellige Parasiten hervorgerufen werden, sind bereits aus der DE 198 25 585 A1 bekannt. Eine bakterielle Wirksamkeit ist bereits in der DE 27 33 658 A1 beschrieben. Auch wenn diese Verbindungen bei der Behandlung von Infektionen, die durch Parasiten oder Bakterien hervorgerufen werden, gute Ergebnisse zeigen, so zeigen auch diese Medikamente unerwünschte Nebenwirkungen.

WO 2002000208 A2 und WO 2021074452 A1 beschreiben beide pharmazeutische Präparate, die als Wirkstoffe 3-N-Formylhydroxylaminopropylphosphonsaurederivate oder 3-N-Acetylhydroxylaminopropylphosphonsaurederivate in Kombination mit Clindamycin oder Artesunat zur Behandlung von Malaria enthalten. WO 2021074452 A1 beschreibt die Akuttherapie bei zerebraler Malaria sowie eine zugehörige Dosierung, insbesondere für die Patientengruppe der Kinder.

WO2012004324 A1 offenbart ebenfalls eine solche Kombination, jedoch in Kombination mit Piperaquine.

In Gegenwart des Standes der Technik ist die Bereitstellung einer Stand-by-Medikation zur Behandlung von Malaria erforderlich.

Die vorliegende Erfindung hat sich daher zur Aufgabe gestellt, die Wirkung dieser pharmazeutischen Mittel für die Stand-by-Medikation zur Behandlung von Malaria bereitzustellen, und zwar bei ersten Symptomen, insbesondere Fieber, solche wie >38,5 °C Körpertemperatur, Schüttelfrost, grippeähnliche Symptome, wie Kopfschmerzen, Muskel- und Gliederschmerzen, Übelkeit und allgemeines Krankheitsgefühl.

Bei Reisen in Malaria-Gebiete wird oft ein Stand-by-Antimalaria-Medikament verschrieben. Das Medikament wird nicht prophylaktisch eingenommen, sondern nur dann, wenn mindestens ein Symptom, insbesondere Fieber auftritt, und keine ärztliche Versorgung verfügbar ist.

Im Stand der Technik ist Malarone^{®} (Atovaquon/Proguanil ) oder Riamet^{®} (Artemether/Lumefantrin ) als Stand-by-Medikamente beschrieben.

Eine Kombination aus Artesunat/Pyronaridin-Atovaquon/Proguanil (APAP) verfügt über eine unabhängige Wirkungsweise von Artesunat, Pyronaridin und Atovaquon-Proguanil, was eine starke Barriere gegen die Entwicklung einer Arzneimittelresistenz darstellen könnte. Pyronaridin wurde als Fixdosistherapie mit Artesunat zur Behandlung der unkomplizierten *P*. *falciparum-Malaria* entwickelt und ist hochwirksam, gut verträglich und sicher (Tona Lutete G, Mombo-Ngoma G, Assi SB, Bigoga JD, Koukouikila-Koussounda F, Ntamabyaliro NY, et al. Pyronaridine-artesunate real-world safety, tolerability, and effectiveness in malaria patients in 5 African countries: A single-arm, open-label, cohort event monitoring studyPLoS Med. 2021;18(6):e1003669). Atovaquon wirkt auf den Cytochrom *bcl-Komplex* von *Plasmodium spp.* und hemmt mehrere Stoffwechselenzyme, wodurch der Parasit inaktiviert wird. Atovaquon-Proguanil zeichnet sich durch eine hohe Wirksamkeit, ein günstiges Verträglichkeits- und Sicherheitsprofil und keine epidemiologisch relevante Arzneimittelresistenz aus. Darüber hinaus sind die Halbwertszeiten der einzelnen APAP-Medikamente relativ gleichmäßig aufeinander abgestimmt.

Jedoch ist an eine Stand-by-Medikation hohe Anforderungen an die Nachhaltigkeit zu stellen, da eine Stand-by-Medikation breit und unspezifisch zur Anwendung kommt und es daher Resistenzen zu vermeiden gilt.

Es wurde nun überraschenderweise gefunden, dass 3-N-Formylhydroxylaminopropylphosphonsäurederivate und / oder 3-N-Acetylhydroxylaminopropylphosphonsäurederivate in Kombination mit Clindamycin und Artesunat - nachstehend "Kombinationspräparat" genannt - insbesondere mit der therapeutischen Anweisung zur Verwendung in der Stand-by-Medikation zur Behandlung von Malaria besonders geeignet ist.

Das erfindungsgemäße Kombinationspräparat, insbesondere Artesunat-Fosmidomycin-Clindamycin hat ebenfalls unabhängige Wirkmechanismen der Einzelsubstanzen, die das Risiko der Selektion von Arzneimittelresistenzen verringern. Während Fosmidomycin selektiv den Nicht-Mevalonat-Weg der Isoprenoid-Synthese hemmt, wirkt Clindamycin direkt auf den Apicoplast von *Plasmodium-Parasiten.* Ein besonders wichtiges Merkmal sind die angepasste Eliminationshalbwertszeiten aller Einzel-Medikamente, nämlich alle kleiner als 5 Stunden. Alle Kombinationspartner sind gut verträglich und sicher. Außerdem hat Fosmidomycin eine nachgewiesene Nebenwirkung auf ein breites Spektrum grampositiver und gramnegativer bakterieller Erreger. Clindamycin wirkt bei mittelschweren bis schweren Infektionen durch grampositive Bakterien und anaerobe Erreger. Das erfindungsgemäße Kombinationspräparat mit antibiotischen Eigenschaften ist klinisch vorteilhaft, da Malaria klinisch nicht von bakteriellen Erkrankungen unterschieden werden kann und es in den am stärksten von Malaria betroffenen Regionen häufig an einer labortechnischen Bestätigung fehlt. Hinzu kommt, dass Patienten mit bakterieller Sepsis in Regionen mit hoher Malariaübertragung häufig gleichzeitig asymptomatische *Plasmodium-Infektionen* aufweisen, die mit Hilfe von Schnelltests leicht erkannt werden und die klinische Diagnose verwirren können. Daher kann das erfindungsgemäße Kombinationspräparat eine starke Anti-Malariawirkung entfalten und gleichzeitig die wichtigsten grampositiven und gramnegativen bakteriellen Erreger von Blutbahninfektionen abdecken.

Während die Eliminationshalbwertszeiten von Atovaquon und Pyronaridin etwa drei Tage bzw. 7-13 Tage betragen, liegen die Eliminationshalbwertszeiten von Artesunat, Fosmidomycin und Clindamycin besonders vorteilhaft *unter fünf Stunden.*

Der Begriff Eliminationshalbwertszeit (auch: Halbwertszeit der Elimination, t_{1/2}) beschreibt in der Pharmakokinetik die Zeitspanne, in der die Konzentration eines Wirkstoffs im Blutplasma auf die Hälfte ihres ursprünglichen Werts sinkt - nach der Phase der vollständigen Aufnahme (Absorption).

Das erfindungsgemäße Kombinationspräparat verhindert besonders vorteilhaft sicher eine Resistenz der Malariaerreger und ist nachhaltig im Fall einer breiten Nutzung, insbesondere bei globalen Reiseaktivitäten, so dass in nachhaltiger Weise keine Arzneimittelresistenz bei Plasmodium auftritt.

Im Rahmen dieser Erfindung wird unter einer "Malaria" eine potenziell lebensbedrohliche Infektionskrankheit verstanden, die durch einzellige Parasiten der Gattung *Plasmodium* verursacht wird. Die Übertragung auf den Menschen erfolgt in der Regel durch den Stich einer infizierten weiblichen Anopheles-Mücke, die während der Blutmahlzeit den Erreger überträgt. Malaria kommt vor allem in tropischen und subtropischen (Reise-)Regionen vor und stellt dort eines der größten gesundheitlichen Probleme dar.

Es gibt fünf Plasmodien-Arten, die beim Menschen Malaria auslösen können. Die bedeutendste ist *Plasmodium falciparum*, die häufigste und gefährlichste Form, die unbehandelt schnell zu schwerwiegenden Komplikationen und sogar zum Tod führen kann. Darüber hinaus existieren die Arten *P. vivax, P. ovale, P. malariae* und *P. knowlesi. P. vivax* und *P. ovale* sind durch die Bildung von Hypnozoiten gekennzeichnet, welche in der Leber ruhende Formen darstellen und zu Rückfällen führen können *P. malariae* kann chronisch verlaufen, während *P*. knowlesi, der vor allem in Südostasien vorkommt, durch eine schnelle Vermehrung ebenfalls schwere Verläufe verursachen kann.

Nach einer Inkubationszeit von etwa sieben bis dreißig Tagen treten meist unspezifische Beschwerden auf. Typisch sind Fieber, Schüttelfrost, Kopfschmerzen, Muskel- und Gliederschmerzen, Übelkeit und allgemeines Krankheitsgefühl.

In schweren Fällen, insbesondere bei *P. falciparum*, können lebensbedrohliche Komplikationen wie Koma, Nierenversagen oder ein multiples Organversagen auftreten. Eine rasche Diagnose ist daher entscheidend. Sie erfolgt in der Regel über einen mikroskopischen Blutausstrich ("Dicker Tropfen") oder mittels Malaria-Schnelltest. In Spezialfällen kommt auch die PCR-Diagnostik zum Einsatz.

Die Behandlung richtet sich nach dem Erregertyp, der Schwere der Erkrankung sowie der Resistenzlage im jeweiligen Infektionsgebiet. Standard ist heute meist eine sogenannte Artemisinin-Kombinationstherapie (ACT). In leichteren Fällen können auch Medikamente wie Atovaquon/Proguanil, Mefloquin oder Chloroquin zum Einsatz kommen. Schwere Fälle erfordern eine stationäre Behandlung, häufig mit intravenösem Artesunat.

Zur Vorbeugung zählt vor allem der konsequente Mückenschutz durch Repellents, das Tragen langer Kleidung und das Schlafen unter imprägnierten Moskitonetzen. In Hochrisikogebieten wird zudem eine medikamentöse Chemoprophylaxe empfohlen. Alternativ kann eine sogenannte Stand-by-Medikation mitgeführt werden, um erfindungsgemäß vorzugsweise bei ersten Symptomen mit dem vorliegenden erfindungsgemäßen Kombinationspräparat, ohne sofortige ärztliche Hilfe eine Behandlung beginnen zu können, welche zudem nachhaltig ist.

Malaria betrifft weltweit jährlich rund 200 bis 250 Millionen Menschen, mit über 600.000 Todesfällen - vor allem bei kleinen Kindern in Afrika südlich der Sahara. Trotz großer Fortschritte in der Bekämpfung bleibt Malaria eine der schwerwiegendsten Infektionskrankheiten weltweit.

Clindamycin bzw. Methyl-6-amino-7-chlor-6,7,8-trideoxy-N-[(2S,4R)-1-methyl-4-propylprolyl]-1-thio-β-L-threo-D-galactooctopyranosid weist die Formel (a) auf:

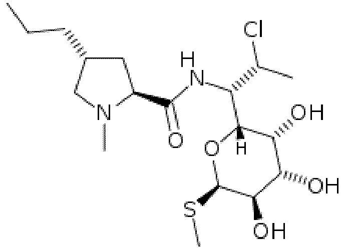

Artesunat bzw. (3R,5aS,6R,8aS,9R,10S, 12R, 12aR)-decahydro-3,6,9-trimethyl-3,12-epoxy-12H-pyrano[4,3-j]-1,2-benzodioxepin-10-ol-hydrogensuccinat weist die Formel (b) auf:

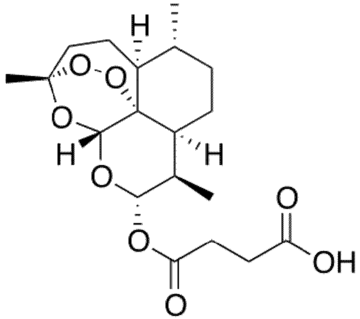

3-N-Formyl-hydroxylamino-propylphosphonsaurederivaten und 3-N-Acetyl-hydroxylamino-propylphosphonsaurederivaten sind erfindungsgemäß Verbindungen der Formel (I) 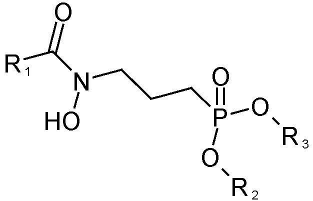
in der R₁ aus der Gruppe ausgewählt ist, die aus Wasserstoff und Methyl besteht,
   und
in der R₂ und R₃ unabhängig voneinander aus der Gruppe ausgewählt sind, die aus Wasserstoff, substituiertem oder unsubstituiertem Alkyl, substituiertem oder unsubstituiertem Acyl, substituiertem oder unsubstituiertem Aryl, substituiertem oder unsubstituiertem Aralkyl, substituiertem oder unsubstituiertem Cycloalkyl, einem substituiertem oder unsubstituiertem Silyl, substituiertem oder unsubstituiertem heterocyclischen Rest besteht, oder zusammen eine substituierte oder unsubstituierte C₁₋₅-Alkylkette bilden, wobei die Alkylgruppen gesättigt oder eine oder mehrere Doppelbindungen oder Dreifachbindungen enthalten können.

Unter diesem Kombinationspräparat sind erfindungsgemäß auch die jeweiligen Salze oder etwaige Stereoisomere zu verstehen und inbegriffen.

Besonders bevorzugt ist, dass R₁, R₂ und R₃ Wasserstoff ist bzw. die Formel I Fosmidomycin ist, einschließlich Salze und Stereoisomere davon.

Weiterhin ist besonders vorteilhaft, dass das erfindungsgemäße Kombinationspräparat auch bei Fieber, welches nicht durch Malaria verursacht wird, die Behandlung in Gegenwart anderer bakteriellen Infektionen erlaubt, da Clindamycin und Formel I, vorzugsweise Fosmidomycin eine breite anti-bakterielle Wirkung aufweisen.

In einer weiteren bevorzugten Ausführungsform beträgt die Dosis zur Verwendung in der Stand-by-Medikation zur Behandlung von Malaria die zwei- bis vierfache Menge (Gewicht in mg) an einem Wirkstoff der Formel I, insbesondere Fosmidomycin gegen Clindamycin und / die zehn-, fünfzehnfache oder zwanzigfache Menge (Gewicht in mg) an einem Wirkstoff der Formel I, insbesondere Fosmidomycin gegen Artesunat.

In einer weiteren bevorzugten Ausführungsform beträgt die Dosis zur Verwendung in der Stand-by-Medikation zur Behandlung von Malaria 1-6 mg Artesunat, 5-15 mg Clindamycin und 10-40 mg gemäß Formel I, insbesondere Fosmidomycin pro kg Körpergewicht eines Patienten, vorzugsweise eine Dosis von 2-4 mg Artesunat, 10 mg Clindamycin und 30 mg gemäß Formel I, insbesondere Fosmidomycin pro kg Körpergewicht eines Patienten.

In einer weiteren bevorzugten Ausführungsform werden diesen Dosen sofort nach dem Auftreten von ersten Symptomen verabreicht.

Weiterhin ist bevorzugt, dass diese Dosen mindestens drei Tage, mindestens zweimal täglich eingenommen werden, insbesondere in einer fixen Kombination mit dem Gesamtgewicht 150 mg, 300 mg oder 600 mg pro Kapsel.

Besonderheiten der obigen Definitionen zur Formel I und geeignete Beispiele dafür werden nachfolgend angegeben:

*"Acyl"* ist ein Substituent, der von einer Säure stammt, wie von einer organischen Carbonsäure, Kohlensäure, Carbaminsäure oder der den einzelnen vorstehenden Säuren entsprechenden Thiosäure oder Imidsäure, oder von einer organischen Sulfonsäure, wobei diese Säuren jeweils aliphatische, aromatische und/oder heterocyclische Gruppen im Molekül umfassen sowie Carbamoyl oder Carbamimidoyl.

Als aliphatische Acylgruppen werden von einer aliphatischen Säure stammende Acylreste bezeichnet, zu denen die folgenden gehören:
Alkanoyl (z.B. Formyl, Acetyl, Propionyl, Butyryl, Isobutyryl, Valeryl, Isovaleryl, Pivaloyl etc.); Alkenoyl (z.B. Acryloyl, Methacryloyl, Crotonoyl etc.);Alkylthioalkanoyl (z.B. Methylthioacetyl, Ethylthioacetyl etc.); Alkansulfonyl (z.B. Mesyl, Ethansulfonyl, Propansulfonyl etc.); Alkoxycarbonyl (z.B. Methoxycarbonyl, Ethoxycarbonyl, Propoxycarbonyl, Isopropoxycarbonyl, Butoxycarbonyl, Isobutoxycarbonyl etc.); Alkylcarbamoyl (z.B. Methylcarbamoyl etc.); (N-Alkyl)-thiocarbamoyl (z.B. (N-Methyl)-thiocarbamoyl etc.); Alkylcarbamimidoyl (z.B. Methylcarbamimidoyl etc.); Oxalo; Alkoxalyl (z.B. Methoxalyl, Ethoxalyl, Propoxalyl etc. ).

Bei den obigen Beispielen für aliphatische Acylgruppen kann der aliphatische Kohlenwasserstoffteil, insbesondere die Alkylgruppe bzw. der Alkanrest, ggf. einen oder mehrere geeignete Substituenten aufweisen, wie Amino, Halogen (z.B. Fluor, Chlor, Brom etc.), Hydroxy, Hydroxyimino, Carboxy, Alkoxy (z.B. Methoxy, Ethoxy, Propoxy etc.), Alkoxycarbonyl, Acylamino (z.B. Benzyloxycarbonylamino etc.), Acyloxy (z.B. Acetoxy, Benzoyloxy etc.) und dergleichen; als bevorzugte aliphatische Acylreste mit solchen Substituenten sind z.B. mit Amino, Carboxy, Amino und Carboxy, Halogen, Acylamino oder dergleichen substituierte Alkanoyle zu nennen.

Als aromatische Acylreste werden solche Acylreste bezeichnet, die von einer Säure mit substituierter oder nicht substituierter Arylgruppe stammen, wobei die Arylgruppe Phenyl, Toluyl, Xylyl, Naphthyl und dergleichen umfassen kann; geeignete Beispiele werden nachfolgend angegeben: Aroyl (z.B. Benzoyl, Toluoyl, Xyloyl, Naphthoyl, Phthaloyl etc.); Aralkanoyl (z.B. Phenylacetyl etc.); Aralkenoyl (z.B. Cinnamoyl etc.); Aryloxyalkanoyl (z.B. Phenoxyacetyl etc.); Arylthioalkanoyl (z.B. Phenylthioacetyl etc.); Arylaminoalkanoyl (z.B. N-Phenylglycyl, etc.); Arensulfonyl (z.B.Benzolsulfonyl, Tosyl bzw. Toluolsulfonyl, Naphthalinsulfonyl etc.); Aryloxycarbonyl (z.B. Phenoxycarbonyl, Naphthyl-oxycarbonyl etc.); Aralkoxycarbonyl (z.B. Benzyloxycarbonyl etc.); Arylcarbamoyl (z.B. Phenylcarbamoyl, Naphthylcarbamoyl etc.); Arylglyoxyloyl (z.B. Phenylglyoxyloyl etc.).

Bei den vorstehenden Beispielen für aromatische Acylgruppen kann der aromatische Kohlenwasserstoffteil und/oder der aliphatische Kohlenwasserstoffteil (insbesondere der Alkanrest) ggf. ein oder mehrere geeignete Substituenten aufweisen, wie solche, die als geeignete Substituenten für die Alkylgruppe bzw. den Alkanrest bereits angegeben sind. Als Beispiel für bevorzugte aromatische Acylreste mit besonderen Substituenten werden mit Halogen und Hydroxy oder mit Halogen und Acyloxy substituiertes Aroyl und mit Hydroxy, Hydroxyimino, Dihalogenalkanoyloxyimino substituiertes Aralkanoyl verstanden sowie Arylthiocarbamoyl (z.B. Phenylthiocarbamoyl etc.); Arylcarbamimidoyl (z.B. Phenylcarbamimidoyl etc.).

Als heterocyclischer Acylrest wird ein Acylrest verstanden, der von einer Säure mit heterocyclischer Gruppe stammt, solche wie folgt:
Heterocyclisches Carbonyl, bei dem der heterocyclische Rest ein aromatischer oder aliphatischer 5-bis 6-gliedriger Heterocyclus mit zumindest einem Heteroatom aus der Gruppe Stickstoff, Sauerstoff und Schwefel ist (z.B. Thiophenyl, Furoyl, Pyrrolcarbonyl, Nicotinoyl etc.);
Heterocyclus-Alkanoyl, bei dem der heterocyclische Rest 5- bis 6-gliedrig ist und zumindest ein Heteroatom aus der Gruppe Stickstoff, Sauerstoff und Schwefel aufweist (z.B. Thiophenyl-acetyl, Furylacetyl, Imidazolylpropionyl, Tetrazolylacetyl, 2-(2-Amino-4-thiazolyl)-2- methoxyiminoacetyl etc. ) und dergleichen.

Bei den obigen Beispielen für heterocyclische Acylreste kann der Heterocyclus und/oder der aliphatische Kohlenwasserstoffteil ggf. einen oder mehrere geeignete Substituenten aufweisen, wie die gleichen, die als geeignet für Alkyl- und Alkangruppen angegeben wurden.

*"Alkylgruppe"* oder *"Alkyl"* ist, soweit nicht anders definiert, ein gerad- oder verzweigtkettiger Alkylrest mit bis zu 26 Kohlenstoffatomen, vorzugsweise jedoch C1-C6 mit Methyl, Ethyl, Propyl, Isopropyl, Butyl, Isobutyl, tert.-Butyl, Pentyl, Hexyl und Struktursomeren davon. Er kann z.B. mit Hydroxy-, Amino-, Halogen- (z.B. Fluor, Brom, Chlor), Oxoresten und Alkoxyresten, wie Methoxy-, Ethoxyresten, substituiert sein.

*"Cycloalkyl"* steht vorzugsweise für ein ggfs. substituiertes C₃₋₈-Cycloalkyl; als mögliche Substituenten sind u.a. Alkoxy (z.B. Methoxy, Ethoxy etc.), Halogen (z.B. Fluor, Chlor, Brom etc.), Nitro und dergleichen geeignet.

*"Aryl"* ist ein aromatischer Kohlenwasserstoffrest, wie Phenyl, Naphthyl usw., der ggf. einen oder mehrere geeignete Substituenten aufweisen kann wie Alkyl, Alkoxy (z.B. Methoxy, Ethoxy etc.), Trifluormethylen, Halogen (z.B. Fluor, Chlor, Brom etc.), Nitro und dergleichen.

Zu *"Aralkyl"* gehören Mono-, Di-, Triphenylalkyle wie Benzyl, Phenethyl, Benzhydryl, Trityl und dergleichen, wobei der aromatische Teil ggf. ein oder mehrere geeignete Substituenten aufweisen kann wie Alkoxy (z.B. Methoxy, Ethoxy etc.), Halogen (z.B. Fluor, Chlor, Brom etc.), Nitro und dergleichen.

Bei der Anwendung der Kombinationstherapie ist es möglich, die Wirkstoffe in einer sogenannten fixen Kombination, d. h. in einer einzigen pharmazeutischen Formulierung zu verabreichen, in der alle drei Wirkstoffe enthalten sind, oder eine sogenannte freie Kombination zu wählen, bei der die Wirkstoffe in Form von getrennten pharmazeutischen Formulierungen gleichzeitig bzw. simultan aber auch nacheinander - zeitversetzt - appliziert werden können.

Das erfindungsgemäße Kombinationspräparat ist gegen Bakterien und ein- und mehrzellige Parasiten wirksam, insbesondere in der Behandlung und Prävention gegen Erreger der Malaria (Plasmodium).

Alle genannten Indikationen samt zugehörigen Symptomen sind im Pschyrembel^{®}, de Gruyter, Berlin beschrieben.

Die pharmazeutisch wirksamen Mittel können in Form von pharmazeutischen Zubereitungen in Dosierungseinheiten zubereitet werden. Dies bedeutet, dass die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt. Tabletten, Dragees, Kapseln, Pillen und Granulate können den oder die Wirkstoffe neben den üblichen Trägerstoffen enthalten, wie a) Füll- und Streckmittel, z.B. Stärken, Milchzucker, Rohrzucker, Glukose, Mannit und Kieselsäure, b) Bindemittel, z.B. Carboxymethylcellulose, Alginate, Gelatine, Polyvinylpyrrolidon, c) Feuchthaltemittel, z.B. Glycerin, d) Sprengmittel, z.B. Agar-Agar, Calciumcarbonat und Natriumcarbonat, e) Lösungsverzögerer, z.B. Paraffin und f) Resorptionsbeschleuniger, z.B. quartäre Ammoniumverbindungen, g) Netzmittel, z.B. Cetylalkohol, Glycerinmonostearat, h) Adsorptionsmittel, z.B. Kaolin und Bentonit und i) Gleitmittel, z.B. Talkum, Calcium- und Magnesiumstearat und feste Polyethylenglykole oder Gemische der unter a) bis i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, dass sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. C14-Alkohol mit C16-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant- Cellulosederivate, Polyethylenglykole, Silikone, Bentonite, Kieselsäure, Talkum und Zinkoxid oder Gemische dieser Stoffe.

Puder und Sprays können neben den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel enthalten.

Lösungen und Emulsionen können neben dem oder den Wirkstoffen die üblichen Trägerstoffe wie Lösungsmittel, Lösungsvermittler und Emulgatoren, z.B. Wasser, physiologische Kochsalzlösung (0,9 %), Ethylalkohol, Isopropylalkohol, Ethylcarbonat, Ethylacetat, Benzylalkohol, Benzylbenzoat, Propylenglykol, 1,3- Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

Das erfindungsgemäße Kombinationspräparat kann parenteral oder oral verabreicht werden. Insbesondere kann die Applikation intravenös (i.v.) erfolgen

Es sei darauf hingewiesen, dass Merkmale, die im Zusammenhang mit einer beispielhaften Ausführungsform oder einem beispielhaften Gegenstand beschrieben werden, mit jeder anderen beispielhaften Ausführungsform oder mit jedem anderen beispielhaften Gegenstand kombiniert werden können.

Wenn ein Begriff mit einem unbestimmten oder bestimmten Artikel, wie zum Beispiel "ein" im Singular bezeichnet wird, schließt dies auch den Begriff im Plural mit ein und umgekehrt, sofern der Kontext nicht eindeutig anderes festlegt.

Der Ausdruck " enthalten(d)", wie er hier verwendet wird, schließt nicht nur die Bedeutung von "umfassen" ein, sondern kann auch "bestehen(d) aus" und "im Wesentlichen bestehend aus" bedeuten.

## Patentansprüche

1. Arzneimittel zur Verwendung in der Stand-by-Medikation zur Behandlung von Malaria enthaltend als Wirkstoff eine Verbindung der allgemeinen Formel I 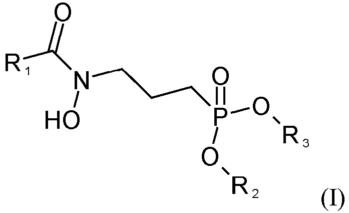
in der R₁ aus der Gruppe ausgewählt ist, die aus Wasserstoff und Methyl besteht, und
in der R₂ und R₃ unabhängig voneinander aus der Gruppe ausgewählt sind, die aus Wasserstoff, substituiertem oder unsubstituiertem Alkyl, substituiertem oder unsubstituiertem Acyl, substituiertem oder unsubstituiertem Aryl, substituiertem oder unsubstituiertem Aralkyl, substituiertem oder unsubstituiertem Cycloalkyl, einem substituiertem oder unsubstituiertem Silyl, substituiertem oder unsubstituiertem heterocyclischen Rest besteht, oder zusammen eine substituierte oder unsubstituierte C₁₋₅-Alkylkette bilden, wobei die Alkylgruppen gesättigt oder eine oder mehrere Doppelbindungen oder Dreifachbindungen enthalten können,
in Kombination mit einem zweiten und dritten pharmazeutischen Mittel, nämlich Clindamycin und Artesunat.

2. Arzneimittel zur Verwendung in der Stand-by-Medikation zur Behandlung von Malaria nach Anspruch 1, **dadurch gekennzeichnet, dass** R₁, R₂ und R₃ jeweils Wasserstoff ist oder die Formel I Fosmidomycin ist.

3. Arzneimittel zur Verwendung in der Stand-by-Medikation zur Behandlung von Malaria nach Anspruch 1 oder Anspruch 2, **dadurch gekennzeichnet, dass** eine Applikation nach dem Auftreten ein oder mehrere Symptome erfolgt.

4. Arzneimittel zur Verwendung in der Stand-by-Medikation zur Behandlung von Malaria nach einem der vorhergehenden Ansprüche, **dadurch gekennzeichnet, dass** eine Applikation nach dem Auftreten ein oder mehrere Symptome sofort erfolgt.

5. Arzneimittel zur Verwendung in der Stand-by-Medikation zur Behandlung von Malaria nach einem der vorhergehenden Ansprüche, **dadurch gekennzeichnet, dass** die Applikation oral oder parenteral, insbesondere intravenös ist.

6. Arzneimittel zur Verwendung in der Stand-by-Medikation zur Behandlung von Malaria nach einem der vorhergehenden Ansprüche, **dadurch gekennzeichnet, dass** mindestens ein oder mehrere Symptome ausgewählt sind aus der Gruppe Fieber, insbesondere >38,5 °C Körpertemperatur, Schüttelfrost, grippeähnliche Symptome, Kopfschmerzen, Muskel- und Gliederschmerzen, Übelkeit, insbesondere Fieber.

7. Arzneimittel zur Verwendung in der Stand-by-Medikation zur Behandlung von Malaria nach einem der vorhergehenden Ansprüche, **dadurch gekennzeichnet, dass** die Eliminationshalbwertszeiten von Artesunat, Fosmidomycin und Clindamycin unter fünf Stunden beträgt.

8. Arzneimittel zur Verwendung in der Stand-by-Medikation zur Behandlung von Malaria nach einem der vorhergehenden Ansprüche, **dadurch gekennzeichnet, dass** keine Arzneimittelresistenz bei Plasmodium auftritt.

9. Arzneimittel zur Verwendung in der Stand-by-Medikation zur Behandlung von Malaria nach einem der vorhergehenden Ansprüche, **dadurch gekennzeichnet, dass** in einer Dosis die zwei- bis vierfache Menge (Gewicht in mg) an einem Wirkstoff der Formel I, insbesondere Fosmidomycin gegen Clindamycin und die zehn-, fünfzehnfache oder zwanzigfache Menge (Gewicht in mg) an einem Wirkstoff der Formel I, insbesondere Fosmidomycin gegen Artesunat vorliegt.

10. Arzneimittel zur Verwendung in der Stand-by-Medikation zur Behandlung von Malaria nach einem der vorhergehenden Ansprüche, **dadurch gekennzeichnet, dass** eine Dosis 1-6 mg Artesunat, 5-15 mg Clindamycin und 10-40 mg gemäß Formel I, insbesondere Fosmidomycin pro kg Körpergewicht eines Patienten beträgt, insbesondere eine Dosis 2-4 mg Artesunat, 10 mg Clindamycin und 30 mg gemäß Formel I, insbesondere Fosmidomycin pro kg Körpergewicht eines Patienten beträgt.
